# EUROPEAN PATENT APPLICATION

(11) **EP 0 523 457 A1**
(43) Date of publication of application: **20.01.1993**
(21) Application number: 92111197.7
(22) Date of filing: 02.07.1992
(51) Int. Cl.: C08J 7/00, C12Q 1/68

(54) **Method for making nylon membrane transparent**

(30) Priority: 02.07.1991 JP 161653/91
(71) Applicant: HITACHI SOFTWARE ENGINEERING CO., LTD., Yokohama-shi, Kanagawa-Ken (JP)
(72) Inventor: Ohgane, Atsushi, Chiba-shi, Chiba-ken (JP); Yamamoto, Kenji, Yokohama-shi, Kanagawa-ken (JP); Yuda, Kouji, Mobara-shi, Chiba-ken (JP); Fujimiya, Hitoshi, Mobara-shi, Chiba-ken (JP); Nasu, Hisanori, Yokohama-shi, Kanagawa-ken (JP)
(74) Representative: Patentanwälte Beetz - Timpe - Siegfried Schmitt-Fumian - Mayr

(57) **Abstract**

Disclosed is the method for making a nylon membrane (12) transparent to thereby allow the sample transcribed on the nylon membrane (12) to be read for the measurement of distribution of the sample with high sensitivity to detection. The nylon membrane (12) on which the sample has been transcribed is made transparent by impregnating the nylon membrane (12) with a clarifying solution (11) containing silicone oil having the composition as follows: 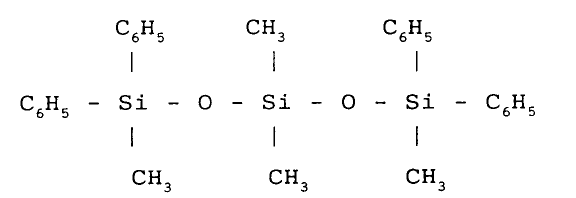
or an isomer or a derivative thereof at the rate of at least 10%.

## Description

### BACKGROUND OF THE INVENTION

The present invention relates to a method for making nylon membrane transparent and, more particularly, to a method for making nylon membrane transparent by clarifying a membrane filter in order to improve a sensitivity to detection in measuring and reading a sample of DNAs and the like transcribed on the membrane filter.

Generally, analysis of structures of various genes for the diagnosis of genetic diseases, amino acids and proteins is carried out by electrophoresis analysis using radioactive isotopes. The electrophoresis analysis is adapted to analyze a sample by electro-phoresing fragments of the sample labelled or replaced with radioactive isotopes in a gel and analyzing a pattern of distribution of the fragments of the sample developed by electrophoresis.

A description will now be made of the diagnosis of genetic diseases as an example of analyzing a sample by reading a pattern of electrophoresis. The human genome consists of DNAs with approximately 3 x 10⁹ pairs of bases. The sequences of the bases are generally constant among the human beings, although there is a deviation in the minute sequences among individuals. This variation in the DNA sequence is called polymorphism of DNAs. Although the DNA polymorphism can be seen not only in a gene region but also in a non-gene region; however, the DNA polymorphism seen in the gene region appears in many occasions as a polymorphism of proteins, which is a phenotype of the DNA polymorphism.

Various types of polymorphisms as shown in the human beings, such as the types of blood, histocompatible antigens, differences in the color of the skin, hairs, etc. among peoples, and the like, are derived from this polymorphism of DNAs. The DNA polymorphism is established by mutation in the DNAs of the gonocyte of the human beings, which has been accumulated in the human beings up to the present time from the time when the human being was developed as an individual biological species in the course of the evolution of the human beings. If this mutation occurs at the site that has the function of significance in the existence as an individual person and when a nosogenic phenotype resulting from the mutation occurs as a pathologic state, the pathologic state is called a genetic disease. It is currently said that there are more than 3,000 kinds of genetic diseases in the human group.

The nosogenesis of the genetic disease is an abnormality appeared in the sequence of DNA. The mutation of the DNA sequence may be recognized for the first time as a disease in several stages: from DNA through mRNA and a protein to a pathogenic phenotype. The diagnosis of a disease is in usual cases carried out in the last stage. If there would be a linear relationship among the disease and the DNAs, proteins, and/or mRNAs as described hereinabove, the diagnosis of the disease can be conducted by analyzing the proteins or DNAs.

The basic technique for the diagnosis of a DNA is based on southern blotting method which basically consists of six steps:
- Step 1:: Extraction of a DNA as a sample;
- Step 2:: Fragmentation of the DNA with restriction enzymes;
- Step 3:: Fractionation of DNA fragments by molecular weights through gel electrophoresis;
- Step 4:: Migration of DNA fragments to a thin layer filter;
- Step 5:: Hybridization of the DNA fractions with a probe DNA (obtained by labelling the DNA having a hemeomorphous sequence of the gene to be detected); and
- Step 6:: Detection of the hybrid by autoradiography.

In diagnosing the genetic disease, DNAs as a sample can be extracted from any organ, whatever it is, and the sample required for this purpose is in usual cases the peripheral blood of the order of several milliliters, from which the leucocyte are separated, followed by separating the DNAs therefrom. Approximately five days are usually required from step 1 to step 6.

For the diagnosis of a genetic disease, a pattern of fractions is provided from a healthy normal person in accordance with the procedures from step 1 to step 6 as described hereinabove and compared with a pattern of fractions obtained from a person to be tested. When it is found that two patterns are the same, the person tested is determined as normal.

The autoradiography to be employed in step 6 presents the problem with safety. In the Southern blotting method, accordingly, the fluorescence method is adopted, in which the probe DNA is labelled with a fluorescent pigment, in place of the radioactive isotopes, and the fluorescent pigment is caused to excite, thereby reading the pattern of fractions.

For the thin layer filter to be employed in step 4, there may usually be employed, for example, nylon membrane or nitrocellulose membrane. If the pattern of the fractions is read from the thin layer filter as it is, the light scattered from the white thin layer filter is so strong that a noise level of the background becomes too high to provide a good S/N ratio because the fluorescence from the probe (the pattern of fluorescence of the sample) is embedded into the noises. Further, even if the surface of the thin layer filter is not flat, the light may scatter from its surface, thereby raising the noise level of the background and as a consequence failing to provide a better S/N ratio. When the quantitative analysis is performed by measuring the percent transmission and the refraction index of the thin layer filter, similar problems may arise because the magnitude of scattering is too strong.

### SUMMARY OF THE INVENTION

The object of the present invention is to provide a method of making nylon membrane transparent for use with measurement of a pattern of a sample with improved sensitivity to detection in reading the sample through the nylon membrane.

In order to achieve the aforesaid object, the present invention consists of the method of making the nylon membrane transparent, which is characterized in that the nylon membrane on which the pattern of the sample is transcribed is made transparent by impregnating it with a clarifying solution containing silicone oil having an optical refraction index which is substantially identical to or similar to that of the nylon membrane.

In the method of making the nylon membrane transparent in accordance with the present invention, the nylon membrane on which the pattern of the sample is transcribed is impregnated with the clarifying solution containing silicone oil having the optical refraction index substantially identical to or similar to that of the nylon membrane, thereby making it transparent. Thereafter, the resulting nylon membrane filter is placed between two sheets of glass support plate panels and the pattern of the sample transcribed on the nylon membrane filter is read. Hence, the scattering light from the surface of the nylon membrane filter can be reduced to a great extent, thereby allowing the pattern of the sample to be read with high sensitivity.

Other objects, features and advantages of the present invention will become apparent in the course of the description of the preferred embodiments, which follows, with reference to the accompanying drawings.

### BRIEF DESCRIPTION OF THE DRAWINGS

Fig. 1 is a schematic representation describing a technique for reading a pattern of electrophoresis of fluorescence type according to an embodiment of the present invention.

Fig. 2 is a schematic representation showing the configuration of the pattern reading system of fluorescence type according to the present invention, which mainly comprises a light collector for receiving fluorescence generated from the thin layer filter and the essential portion of the optic system of an optoelectrical conversion unit.

### DETAILED DESCRIPTION OF THE PREFERRED EMBODIMENTS

A description will be made of an embodiment of the present invention with reference to the accompanying drawings.

In this embodiment according to the present invention, there is employed nylon membrane filter as the thin layer filter for reading a pattern of electrophoresis on the thin layer filter on which DNAs of a sample are transcribed. The procedures for transcribing the DNAs on the thin layer filter involve separating a gel containing a sample labelled with no fluorescent pigment by electrophoresis, placing a thin layer filter on the gel after the completion of the electrophoresis, and drawing up the DNA sample from the gel into the thin layer filter by taking advantage of the same principle as the electrophoresis. As the thin layer filter to be employed in this embodiment, there may be employed nylon membranes such as membranes of nylon 6, nylon 66 and the like, which in turn is subjected to surface treatment in order to allow the thin layer filter to readily adsorb the DNA sample. Thereafter, the sample is labelled with a substance, referred to as a probe, which is likely to be bound to the sample transcribed on the thin layer filter and on which a fluorescent substance is adsorbed, followed by reading a pattern of distribution of the fluorescent substance adsorbed on the sample.

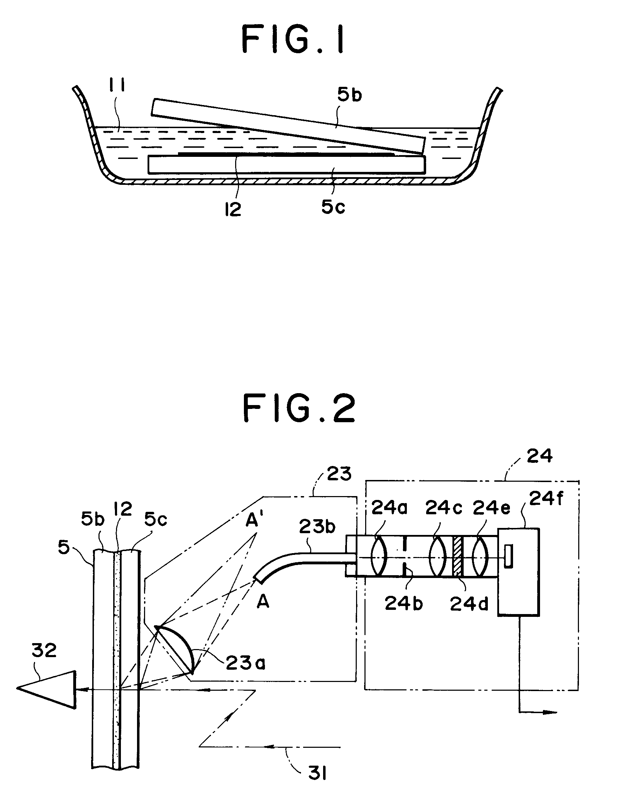

Fig. 1 is a schematic representation describing the technique for reading the pattern of electrophoresis of fluorescence type according to an embodiment of the present invention. The DNA sample which has been transcribed on the thin layer filter and then labelled with the fluorescent probe can be read directly from the thin layer filter by means of a fluorescent pattern reading apparatus. It is noted, however, that the thin layer filter itself is so white in color that the scattering light is strong and as a consequence the sensitivity to detection of the fluorescence is lowered, for example, by one digit than when the DNA sample is read directly from the gel. Hence, this technique may suffer from the difficulty of reading the DNA sample having low intensity of fluorescence.

Next, a description will be made of the procedures of treating the sample for restricting the intensity of scattering light to a low level. The procedures involve transcribing the electrophoresed DNA sample on the thin layer filter, adsorbing the probe labelled with the fluorescent substance on the DNA sample transcribed on the thin layer filter, immersing the thin layer filter in a clarifying solution 11, on which the DNA sample is transcribed and the fluorescent substance is adsorbed so as to emit fluorescence from the DNA sample labelled with the fluorescent probe, as shown in Fig. 1, and placing the thin layer filter 12 between glass support plate panels 5b, 5c. The glass support plate panels 5b, 5c serve as making the surface of the thin layer filter 12 flat and play a role for reducing the amount of light noises due to scattering. Careful attention should be paid in such a manner that bubbles do not enter into a gap between the glass support plate panels as much as possible in placing the thin layer filter 12 between the glass support plate panels 5b, 5c. For the clarifying solution 11, there may be employed any one as long as it has an index of optical refraction which is substantially identical to or similar to that of the thin layer filter 12 and it does not adversely affect the DNA sample. For example, when the thin layer filter made of a material such as nylon 66 or nylon 6 is employed as the thin layer filter 12, the thin layer filter has a mean reflection index of approximately 1.55, although it depends upon the orientation of the molecules, so that a solution having the refraction index in the range from 1.52 to 1.57 is suitable for the clarifying solution 11. At this end, silicone oil having the following composition may preferably be employed: 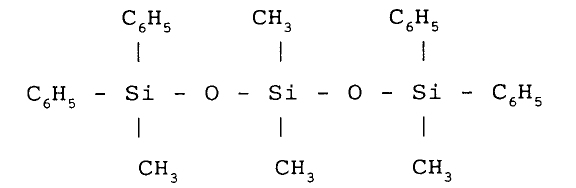
Further, nylon 11, nylon 12 and the like may be employed as the thin layer filter 12. As the glass support plate panels 5b, 5c, there may be employed boron silicate glass which generates comparatively less fluorescence that acts as noises in reading the pattern of fluorescence. In addition, for example, quartz glass and various optical glass may be employed as the glass support plate panels 5b, 5c.

As the thin layer filter 12 is composed of a material having a large number of fine pores, the impregnation of the thin layer filter 12 with the clarifying solution 11 covers fine pores in the thin layer filter 12 with the clarifying solution 11, thereby making the thin layer filter 12 transparent and reducing the level of scattering light to a great extent. Further, by placing the thin layer filter 12 between the glass support plate panels 5b, 5c, the clarifying solution 11 is allowed to infiltrate uniformly into the thin layer filter 12 and the surface of the thin layer filter 12 is made optically uniform, thereby further reducing the level of scattering light. The thin layer filter 12 placed and interposed between the glass support plate panels 5b, 5c is set in the fluorescent pattern reading apparatus to read the pattern of fluorescence in the thin layer filter.

Fig. 2 is a schematic representation showing the configuration of the pattern reading system of fluorescence type according to the present invention, which mainly comprises a light collector for receiving fluorescence generated from the thin layer filter and the essential portion of the optic system of an optoelectrical conversion unit. As shown in Fig. 2, the pattern reading system is provided with a sample reading unit 5 where the thin layer filter 12 placed and interposed between the glass support plate panels 5b, 5c is set. The thin layer filter 12 is irradiated with laser beams 31 as irradiating light, which is so arranged as to move along and scan the thin layer filter 12. The laser beams 31 penetrate through the glass support plate panels 5b, 5c in their thickness direction and reach the thin layer filter 12 which has been made transparent. The laser beams 31 further advances into and through the thin layer filter 12 in its thickness direction. After they have penetrated through the thin layer filter 12, the laser beams 31 are arranged to enter into a light trap 32 and then damped down so as not to adversely affect the reading system as stray light. Fluorescence 13 from the sample reading unit 5 and scattering light of exciting light, generated from the surfaces of the glass support plate panels 5b, 5c, are arranged to reach a cylindrical glass 23a and they are allowed to form image on the opposite side of the cylindrical glass 23a as shown in Fig. 2. In Fig. 2, the point A is the focal point where the fluorescence from the thin layer filter 12 and the scattering light of the exciting light generated from the thin layer filter 12 meet together. For the scattering light of the exciting light generated from the surface of the glass support plate panels 5b, 5c, for example, the scattering light of the exciting light from the glass support plate panel 5b is arranged so as to meet together at the focal point A' in Fig. 2. An optical fiber array 23b is disposed in the position so as to receive the fluorescence from the thin layer filter 12 at the focal point A and form the image at the focal point A. The relationship of the spatial positions of a light receiving path allows the fluorescence to be separated from the scattering light from the glass support plate panels 5b, 5c in an optogeometrical way.

The fluorescence collected by the optical fiber array 23b is led through an optical fiber to an optoelectrical conversion unit 24. The fluorescence that entered into the optoelectrical conversion unit 23 is processed to extract its parallel components only by using a first lens 24a, a restrictor 24b, and a second lens 24c, and the parallel components are entered into an optical filter 24d. After only the scattering light has been removed by the optical filter 24d, the fluorescence is collected by a third lens 24e and then led to a photomultiplier tube 24f where the fluorescence detected is converted into electric signals. In the optoelectrical conversion unit 24, only the parallel light components of the entering light is extracted by the second lens 24c and entered into the optical filter 24d at a right angle in order to improve the performance for separating wavelengths of the optical filter 24d. On the other hand, the scattering light of the exciting light generated from the surface of the thin layer filter 12 is separated by the optical filter 24d in order to improve a signal-to-noise ratio, and the fluorescence separated from the scattering light is then collected by the third lens 24e and led to the photomultiplier tube 24f.

As described hereinabove, after it has been received and collected by the optical fiber array 23b and the scattering light has been removed therefrom by the optical filter 24d, the fluorescence led to the photomultiplier tube 24f is then converted into electric signals which in turn are processed into image data, thereby providing the objective image of electrophoresis.

In accordance with the present invention, the thin layer filter is made transparent by impregnating it with a solution having an optical index of refraction substantially identical to or similar to that of the thin layer filter and then it is read in a state that it is placed or interposed between transparent glass support plate panels. This arrangement can reduce the level of scattering light generated from the thin layer filter so that the pattern of fluorescence on the thin layer filter can be read in a high signal-to-noise ratio.

## Claims

1. A method for making a nylon membrane (12) transparent, wherein the nylon membrane on which a sample DNA has been transcribed is impregnated with a clarifying solution (11) containing silicone oil having an index of optical refraction substantially identical to that of the nylon membrane (12).

2. A method as claimed in claim 1, wherein said clarifying solution (11) contains silicone oil having the composition as follows: 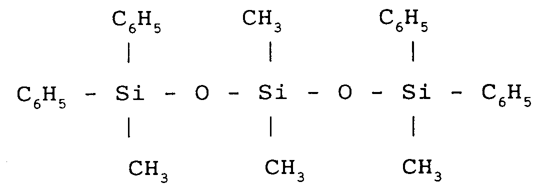 or an isomer or a derivative thereof at the rate of at least 10%.

3. A method as claimed in claim 1, wherein said nylon membrane with said sample DNA transcribed thereon is impregnated with the clarifying solution (11) having the index of optical refraction substantially identical to or similar to said nylon membrane (12), it is placed or interposed between sheets of transparent glass panels (5b, 5c) and a pattern of fluorescence is read.

4. A method as claimed in claim 3, wherein said transparent glass panel (5b, 5c) is a boron silicate glass plate.

5. A method as claimed in claim 1, wherein said nylon membrane (12) is a nylon membrane filter (12) made of nylon 6 or nylon 66 and the index of optical reference of the clarifying solution (11) containing silicone oil is in the range of from 1.52 to 1.57.
